# EUROPÄISCHE PATENTANMELDUNG

(11) **EP 1 759 706 A2**
(43) Veröffentlichungstag der Anmeldung: **07.03.2007**
(21) Anmeldenummer: 06120083.8
(22) Anmeldetag: 04.09.2006
(51) Int. Cl.: A61K 36/515

(54) **Verwendung von Enzianwurzel-Maische und entsprechende Mittel**

(30) Priorität: 05.09.2005 DE 102005042139
(71) Anmelder: Priebe, Ingrid, 83471 Schönau (DE)
(72) Erfinder: Priebe, Ingrid, 83471 Schönau (DE)
(74) Vertreter: Reitstötter - Kinzebach

(57) **Zusammenfassung**

Die vorliegende Erfindung betrifft die Verwendung vergorener Enzianwurzel-Maische zu kosmetischen und therapeutischen Zwecken sowie Mittel auf Basis vergorener Enzianwurzel-Maische.

## Beschreibung

Die vorliegende Erfindung betrifft die Verwendung vergorener Enzianwurzel-Maische zu kosmetischen und therapeutischen Zwecken sowie Mittel auf Basis vergorener Enzianwurzel-Maische.

Erhebliche soziale und wirtschaftliche Bedeutung kommt heute unspezifischen Unwohlseins- und Erschöpfungszuständen zu, teils durch Stress und/oder anderweitig ungesunde Lebensführung bedingt, teils unklarer oder komplexer Ätiologie. Es handelt sich hierbei um Beeinträchtigungen des subjektiven Wohlbefindens, die mit objektiven medizinischen Erkrankungen einhergehen können, aber nicht müssen, und deren Behandlung daher teils kosmetisch, teils medizinisch ist. Großer Beliebtheit erfreuen sich daher sogenannte "Wellness"-Programme, die durch a-däquate Maßnahmen, zu denen auch medizinische Behandlungen gehören können, das subjektive Wohlbefinden zu verbessern versuchen.

In den letzten Jahren haben die Möglichkeiten der "natürlichen" Therapie- und Behandlungsformen insbesondere der genannten Wohlbefindensstörungen, aber auch chronischer Erkrankungen, zunehmende Aufmerksamkeit weiter Kreise auf sich gezogen. Speziell die Phytotherapie, deren medizinischer wie kosmetischer Nutzen sich weitgehend objektivieren lässt, erfreut sich zunehmender Beliebtheit, entweder als Einzelbehandlung oder in Kombination mit anderen Maßnahmen bis hin zur "ganzheitlichen Medizin", die dem Patienten bzw. Kunden ein in sich schlüssiges Paket an trotz des Namens kosmetischen und/oder medizinischen Maßnahmen anzubieten versucht. Gerade im heutigen "Wellness"-Bereich werden gerne phytotherapeutische, mechanische (insbesondere Massagen), balneotherapeutische und sportliche Maßnahmen und eventuell auch medizinische Behandlungen zur Verbesserung des gesamten Wohlbefindens einer Person kombiniert.

Bei der phytotherapeutischen Komponente solcher Behandlungen wird hierbei insbesondere gerne auf als traditionell geltende Heilpflanzen einschließlich der "Volksmedizin" verschiedener Nationen zurückgegriffen, wobei der Begriff "Heilpflanzen" auch Teile oder Produkte von bekannten Nahrungsmitteln umfasst. So beschreibt US2005/0191268 die Verwendung von bei der Weinherstellung anfallenden Pressrückständen zur Herstellung kosmetischer Zubereitungen.

Schon lange vor dem Aufkommen der modernen Wissenschaft waren Pflanzen der Gattung Enzian (Gentiana), insbesondere der Gelbe Enzian (Gentiana lutea), über die Grenzen ihres natürlichen Verbreitungsgebietes (Gebirge Süd- und Südmitteleuropas einschließlich der Alpen und Pyrenäen) hinaus für ihren Reichtum an geschmacksaktiven und pharmakologisch wirksamen Inhaltsstoffen bekannt. Enziane wurden über die Jahrhunderte hinweg sowohl nahrungsmitteltechnisch, vor allem in Form von Spirituosen (Enzianschnäpse, oft auch "Alpenbitter" oder "Enzler" genannt), daneben auch als Gewürze bzw. bittere Gewürzbestandteile für Speisen und Getränke, wie etwa in DE3006765 beschrieben, als auch in traditionellen pharmazeutischen Zubereitungen extensiv genutzt, wobei die letzteren vom mittelalterlichen Universalheilmittel Theriak bis zu homöopathischen Anwendungen reichten. Im Bereich der Nahrungsmittel ist die Verwendung als Würzmittel in unserer Zeit aufgrund von Änderung der Geschmacksvorlieben und Verfügbarkeit billigerer Importgewürze gegenüber der Schnapsbrennerei in den Hintergrund getreten; auf dem pharmazeutischen Sektor ist Radix Gentianae nach wie vor eine geschätzte pflanzliche Droge.

Die Inhaltsstoffe von Radix Gentianae, deren Gehalt jahreszeitlich schwankt und wenigstens teilweise zur Höhenlage des Standortes direkt proportional ist, umfassen außer verschiedenen Oligo- und Polysacchariden und ätherischen Ölen vor allem Xanthone und Xanthonglucoside, Flavonoide wie Gentisin und Flavonoidglykoside sowie Saponine, daneben zur Gruppe der Secoiridoidglykoside gehörige Monoterpene (insbesondere Gentiopicrosid) und davon abgeleitete Alkaloide, die als Gentianaalkaloide bekannt sind. Verwendung findet die Droge in erster Linie als alkoholische Tinktur bzw. Extrakt und gelegentlich auch in Teezubereitungen, aber auch (in Japan) als Trockenpulver und sogar (in Großbritannien) nach entsprechender Aufbereitung als Infusionslösung. Ihre Wirkungen sind als sekretionsfördernd, vor allem mit Wirkung auf Speicheldrüsen und Magensaftbildung, und allgemein sekretolytisch beschrieben worden. Ferner wurde bei neueren Untersuchungen auch eine deutliche Verringerung der Sekretion von IgA beobachtet, die bei chronisch entzündlichen Krankheiten wie dem Morbus Crohn / Colitis ulcerosa-Komplex als günstig gilt. Weiterhin wurde eine ausgeprägte antimikrobielle, speziell fungizide, Wirkung beschrieben, überdies auch eine heilsame Wirkung bei Protozoonosen wie den schwer zu behandelnden Leishmania-Infektionen. Schließlich deuten neuere Befunde auch auf zentralnervöse Wirkungen hin; speziell die Gentianaalkaloide steigern die Wirkung von Schmerzmitteln und besitzen blutdrucksenkende, entzündungshemmende und muskelrelaxierende Wirkungen, während Gentisinsäure leicht fiebersenkend wirkt.

Anwendung findet Radix Gentianae dementsprechend in erster Linie bei Magen- und Leberleiden, insbesondere zur Steigerung der Gallensekretion und bei Appetitlosigkeit, bei Gicht, bei Beschwerden im Nieren- und Harnwegssystem und weiblichen Beschwerden, gelegentlich auch zur Behandlung von Atemwegserkrankungen und Erkrankungen im Mund- und Rachenraum, ferner zur Behandlung von Prellungen, Verstauchungen und Verbrennungen ebenso wie von Insektenstichen und anderen Hautleiden, aber auch bei Befindlichkeitsstörungen wie Schlafstörungen, Verspannungen, Nervosität und von chronischen und/oder undefinierten Schmerzen, weiterhin bei entzündlichen Vorgängen im weitesten Sinne einschließlich von rheumatoiden und degenerativen Erkrankungen und schließlich von Ödemen.

Die geläufige Darreichung von Radix Gentianae erfolgt systemisch, mit Ausnahme der wenig verwendeten Infusionslösung durchweg oral. Für die topische Anwendung wurden bislang nur unvergorene Extrakte in Betracht gezogen, so beschreibt US 5,595,743 eine Kräutermedizin mit einem Gehalt von bis zu 40 % eines enzymatisch aufgeschlossenen, jedoch unvergorenen Enzianextraktes. Ferner beschreibt AT 289686 einen Kräuteressig auf Weinessig-Basis mit einem Anteil von 3 % eines unvergorenen wässrigen Kräuterextraktes, z. B. Enzianextraktes, als Nahrungs- und Kosmetikzusatz.

Eine Übersicht über die pharmazeutischen Aspekte von Drogen aus Gewächsen der Gattung Gentiana findet sich in Hagers Handbuch der pharmazeutischen Praxis, 5. Auflage, Band 5, 227-247 (1993). Die Verwendung von Enzian als Bitterstoff zur Herstellung von leistungssteigernden und verdauungsanregenden Schnäpsen wird in Th. Brendler et al., Heilpflanzen, und in G. Madaus, Lehrbuch der biologischen Heilmittel, Bd. II, angeführt.

Der Reichtum der Enzianwurzel an Bitterstoffen, der ihre Verwendung als Würzmittel und Spirituosengrundlage bedingt, ist zugleich als Hauptnachteil bei ihrer heutigen phytotherapeutischen Verwendung anzusehen: Während in früheren Jahrhunderten der unvermeidliche Bittergeschmack generell mit medizinischer Wirksamkeit assoziiert wurde und dadurch unmittelbar zu einem subjektiven Empfinden von "Heilung" führte, das die psychische Wahrnehmung der Beschwerden linderte und die objektive Heilung fördern konnte, ist die heutige subjektive Wahrnehmung bitterer Geschmäcke mit "Medizin" und "Kranksein" assoziiert und damit subjektiv und objektiv kontraproduktiv, insbesondere, wo es um die im wesentlichen palliative Behandlung leichterer, aber chronischer Beschwerden geht, und noch mehr in den Bereichen Kosmetik, Entspannung und Wellness, in denen ein unangenehmer oder Widerwillen erregender Geschmack nicht akzeptabel ist. Hinzu kommt möglicherweise noch eine größere unmittelbare Empfindlichkeit gegenüber Bitterstoffen aufgrund des fast vollständigen Fehlens solcher in der modernen Ernährung.

Aufgabe der vorliegenden Erfindung war es also, eine weitere Möglichkeit bereitzustellen, den Enzian für Anwendungen im Kosmetik- und "Wellness"-Bereich als auch in der Behandlung von leichteren Beschwerden nutzbar zu machen.

Überraschenderweise wurde nun gefunden, dass eine topische Applikation eines Mittels auf der Basis vergorener Enzianwurzel-Maische sowohl für kosmetische als auch therapeutische Anwendungen möglich und sinnvoll ist.

Ein Gegenstand der vorliegenden Erfindung ist daher ein pharmazeutisches oder kosmetisches topisches Mittel, das durch einen Gehalt an vergorener Enzianwurzel-Maische gekennzeichnet ist.

Ein weiterer Gegenstand der vorliegenden Erfindung ist die Verwendung einer vergorenen Enzianwurzel-Maische zur Erhöhung des Wohlbefindens beziehungsweise zur Linderung von Beschwerden einer Person.

Als Lieferanten für geeignete Enzianwurzeln kommen hierbei grundsätzlich die folgenden der über 200 Angehörigen der Gattung Gentiana Linné und alle ihre Unterarten und Varietäten in Betracht: G. asclepiadea (Schwalbenwurzenzian), G. cruciata (Kreuzenzian), G. lutea (Gelber Enzian), G. pannonica (Ungarischer Enzian), G. punctata (Gefleckter Enzian), G. scabra (Japanischer Enzian), G. purpurea (Purpurenzian), G. manshurica (Mandschurischer Enzian), G. regescens, G. triflorica (Dreiblütiger Enzian), G. dahurica, G. macrophylla (Großblattenzian), G. crassicaulis (Dickstammenzian), G. straminae, G. quinquefolia (Fünfblattenzian) und andere. Aufgrund der Größe des Rhizoms und der Möglichkeit des gezielten Anbaus zur Deckung des Bedarfs ist hierbei G. lutea bevorzugt.

Vergorene Enzianwurzel-Maische ist in herkömmlicher Art und Weise erhältlich. Insbesondere fällt eine solche Maische bei Herstellung von entsprechenden Spirituosen an. Die jeweils anwendbaren Techniken des Einmaischens, der Vergärung und weiterer Verfahrensschritte sind auf dem Gebiet der Spirituosenherstellung geläufig, ebenso der Anbau und die Ernte von Enzianwurzeln.

In einer Ausführungsform der Erfindung ist das Enzianwurzelprodukt erhältlich, indem man Enzianwurzeln mechanisch aufschließt, mit Wasser versetzt, vergärt und gegebenenfalls wenigstens einen Teil des gebildeten Alkohols abtrennt.

In der Regel werden zuvor auf ihre Qualität geprüfte Enzianwurzeln eingesetzt. Zur Wertminderung können hierbei Fermentierungsprozesse in zu lange gelagerten Wurzeln sowie Verwechselungen mit dem vegetativ ähnlichen Germer (Veratrum album) beitragen; Überprüfung erfolgt anhand der in den jeweils gültigen Arzneibüchern bzw. Pharmakopöen (z.B. Ph. Eur., Europäisches Arzneibuch 2004 - Amtliche Ausgabe, ISBN 3-7692-3783-8) empfohlenen Maßnahmen. So kann man prinzipiell auch getrocknete Wurzeln verwenden, wobei ein rascher Trocknungsvorgang zweckmäßig ist. Die Verwendung von Frischwurzeln stellt einen besonderen Aspekt der vorliegenden Erfindung dar. Zu beachten ist hierbei, dass sich die Zusammensetzung der Wurzeln je nach Alter und Trocknungsgrad ändern kann. Frische Wurzeln enthalten in der Regel etwa 50 bis 80 Gew.-%, beispielsweise etwa 70 Gew.-% Wasser und 5 bis 13 Gew.-% Zucker; bei getrocknetem Material kann der Zuckergehalt auf 20 bis 40 Gew.-%, in der Regel bis etwa 30 Gew.-% ansteigen.

Ferner ist es Vorteil, die Wurzeln vor dem Aufschließen zu waschen, beispielsweise mit Wasser.

Das mechanische Aufschließen kann zweckmäßigerweise durch Zerkleinern erfolgen. Dazu kann man die Wurzeln, erforderlichenfalls unter Verwendung geeigneter Vorrichtungen, zerschneiden, zerhacken oder andere geeignete Maßnahmen ergreifen, die einen hinreichenden Aufschluss der Wurzeln gewährleisten. Vorzugsweise erfolgt das Zerkleinern mit Hilfe eines Häckslers.

Das aus dem Aufschluss resultierende Wurzelprodukt, in der Regel ein grob- bis feinkörniges Pulver, wird dann zur Vergärung mit Wasser versetzt, wobei man pro 1 kg Wurzelprodukt in der Regel etwa 0,5 bis 10 l Wasser, bevorzugt etwa 1 bis 5 l Wasser und besonders bevorzugt etwa 2 l Wasser einsetzt. Diese Mengen haben sich vor allem dann als zweckmäßig erwiesen, wenn die Gesamtansatzmenge im Bereich von 1000 bis 10000 kg Wurzelprodukt liegt.

Der Begriff "Vergärung" bezeichnet hierbei eine Umsetzung, bei der wenigstens ein Teil der im Pflanzenmaterial enthaltenen Zucker, insbesondere der niedermolekularen Zucker (Oligosaccharide), z. B. der Gentianose, in Alkohole umgewandelt wird.

Die Vergärung basiert im Wesentlichen auf dem als alkoholische Gärung bezeichneten Vorgang, bei dem Zucker in Alkohol und Kohlendioxid umgewandelt wird. Die Vergärung des Enzianwurzel-Wasser-Gemischs erfolgt vorzugsweise durch Hefe, besonders bevorzugt Bierhefe (Saccharomyces cerevisiae), auch Bäckerhefe genannt, oder durch aktive Trockenhefe bzw. zellfreie Hefeextrakte. Dabei liegt die Menge der zu verwendenden Bäckerhefe bezogen auf die Menge Wasser in der Regel in einem Bereich von etwa 1 g/l bis 50 g/l, bevorzugt etwa 2 g/l bis 25 g/l, besonders bevorzugt etwa 5 g/l bis 15 g/l. Bezogen auf die Menge des Enzianwurzelprodukts liegt die Menge der zu verwendenden Bäckerhefe in der Regel in einem Bereich von etwa 1 g/kg bis 100 g/kg, bevorzugt etwa 5 g/kg bis 50 g/kg, besonders bevorzugt etwa 15 g/kg bis 25 g/kg. Andere Hefen, aktive Trockenhefe oder zellfreie Hefeextrakte werden in einer der Aktivität der Bäckerhefe entsprechenden Menge eingesetzt. Außerdem können weitere Zusätze, beispielsweise Ammoniumsulfat zur Vermeidung von Gärhemmungen, zweckmäßig sein.

Das Wurzelprodukt, Wasser und Hefe umfassende Gemisch wird erfindungsgemäß als Enzianwurzel-Maische bezeichnet. In einer besonderen Ausführungsform ist die vergorene Enzianwurzel-Maische aus einer Enzianwurzel-Maische erhältlich, die im Wesentlichen aus Wurzelprodukt, Hefe und Wasser besteht.

Die Gärtemperatur beträgt in der Regel etwa 15°C bis 40°C. Bevorzugt ist eine Gärtemperatur im Bereich von 25°C bis 35°C beträgt, besonders bevorzugt etwa 30°C. Enzianwurzeln benötigen eine relativ lange Gärzeit, da der hauptsächlich vorhandene Zucker, das Trisaccharid Gentianose, nicht direkt vergärbar ist und daher zunächst enzymatisch in Glucose und Fructose aufgespaltet werden muss. Gärzeiten im Bereich von mehreren Wochen, in der Regel 2 bis 10 Wochen, sind üblich.

Eine erfindungsgemäße vergorene Enzianwurzel-Maische liegt auch dann vor, wenn nicht der gesamte Zucker in Alkohol überführt ist. Üblicherweise kommt die Vergärung mit abnehmendem Zuckergehalt und ansteigender Alkoholkonzentration allmählich zum Stillstand, was sich an der asymptotisch gegen Null gehenden Kohlendioxid-Entwicklung ablesen lässt. So ist einer besonderen Ausführungsform zufolge die vergorene Enzianwurzel-Maische durch im Wesentlichen vollständige Vergärung erhältlich, womit eine unter den gewählten Bedingungen im Wesentlichen zum Stillstand gekommene Vergärung gemeint ist.

Im Anschluss an die Vergärung kann man gegebenenfalls wenigstens einen Teil des gebildeten Alkohols abtrennen. Somit kann man erfindungsgemäß sowohl die aus dem Gärungsprozess vor dem Abtrennen des gebildeten Alkohols (ungebrannte Maische) als auch die nach Abbrennen wenigstens eines Teils des gebildeten Alkohols erhältliche Maische (gebrannte Maische) verwenden.

Das Abtrennen des Alkohols kann in herkömmlicher Art und Weise erfolgen und beinhaltet in der Regel ein Abdestillieren (dem "Abbrennen") des für die Spirituosenherstellung verwertbaren Teils der vergorenen Maische. Der nach dem Abtrennen anfallende Rückstand, eine erfindungsgemäße vergorene Enzianwurzel-Maische, ist unter dem Begriff "Schlempe" bekannt.

Sowohl die vor als auch die nach dem Abtrennen von Alkohol erhältlichen Maischen, Schlempe eingeschlossen, liegen in der Regel in Form einer wässrigen Suspension, sprich eines Breis, vor. Da sich ein solcher Brei hervorragend zur erfindungsgemäßen Anwendung eignet, können die mit obigen Verfahren erhältlichen Maischen bzw. Schlempen direkt verwendet werden. Alternativ kann man sie zunächst trocknen und vor der Verwendung in eine anwendungsfreundliche Form überführen, beispielsweise in Wasser suspendieren. Zur Trocknung eignet sich besonders die alkoholabgereicherte Schlempe. Weiterhin können Grobbestandteile vor oder nach dem Abbrennen durch Filtration aus der Maische bzw. Schlempe entfernt werden, so dass Maische bzw. Schlempe mit einer eher flüssigen als breiartigen Konsistenz erhalten wird. Da die für die erfindungsgemäße Verwendung wichtigen Inhaltsstoffe in der Hauptsache in der flüssigen Phase gelöst oder dispergiert vorliegen, ist es sogar möglich, die festen Bestandteile nahezu vollständig abzutrennen. Das resultierende, mehr einer Lösung oder Dispersion als einer Suspension entsprechende Produkt gehört ebenfalls zu den erfindungsgemäßen Maischen bzw. Schlempen.

In einer bevorzugten Ausführungsform ist der Alkoholgehalt des fertigen Produktes gering. Er liegt vorzugsweise unter 15 Gew.-%, besonders bevorzugt unter 5 Gew.-%, z. B. unter 1 Gew.-%, jeweils bezogen auf das Gesamtgewicht des fertigen Produktes.

Die Verwendung einer vergorenen Enzianwurzel-Maische zur Erhöhung des Wohlbefindens einer Person betrifft auch an sich gesunde Personen und findet insbesondere im "Wellness"-Bereich Anwendung. Eine Erhöhung des Wohlbefindens beruht in der Regel auf subjektiver Wahrnehmung der behandelten Person und ist häufig allgemeiner Art. Hierbei sind unter "Steigerung des allgemeinen Wohlbefindens einer Person" außer den geläufigen kosmetischen Maßnahmen auch alle Maßnahmen gegen unspezifische Erschöpfungs- und/oder Unwohlseinszustände zu verstehen. Eine Erhöhung des Wohlbefindens äußert sich im kosmetischen Bereich insbesondere durch eine als entspannend, entstauend und/oder die Haut glättend empfundene Wirkung und im Übrigen durch eine Besserung besagter unspezifischer Erschöpfungs- und/oder Unwohlseinszustände.

Die Verwendung einer vergorenen Enzianwurzel-Maische zur Linderung von Beschwerden einer Person betrifft hingegen eine therapeutische Behandlung, worunter auch palliative und/oder kurative Maßnahmen bei objektiv feststellbaren Krankheitsbildern zu verstehen sind. Hierzu gehören insbesondere Maßnahmen bei folgenden Beschwerden:
- Stoffwechselstörungen, namentlich
   o Gicht;
- Beschwerden im Nieren- und Harnwegssystem;
- Beschwerden im Verdauungstrakt, namentlich
   o Sodbrennen,
   o Verdauungsschwäche und
   o Verstopfung;
- Bindegewebsschwäche;
- Verletzungen, namentlich
   o Prellungen,
   o Verstauchungen,
   o Verbrennungen und
   o Insektenstichen;
- Befindlichkeitsstörungen, namentlich
   o Schlafstörungen,
   o Verspannungen,
   o Schwindel,
   o Nervosität und
   o chronischen und/oder undefinierten Schmerzen;
- entzündlichen Vorgängen, namentlich
   o Rheuma,
   o Arthrose,
   o Arthritiden,
   o Phlebitiden,
   o chronischen Darmentzündungen und
   o Multipler Sklerose;
- Menstruationsbeschwerden;
- Herz-/Kreislaufbeschwerden, Krampfadern, Durchblutungsstörungen und Blutarmut;
- Hauterkrankungen, namentlich
   o Neurodermitis; und
- Lymphstauungen.

Erfindungsgemäß bevorzugt ist die Verwendung in Form einer topischen Anwendung. Eine solche erlaubt in besonderem Maße die Behandlung von Hautleiden einschließlich der zur Steigerung des allgemeinen Wohlbefindens zu rechnenden kosmetischen Behandlung von Falten oder allgemein alternder oder ästhetisch unschöner Haut sowie die Linderung von Beschwerden im Zusammenhang mit Lymphstauungen, die sowohl kosmetisch als auch medizinisch sein kann. Gemäß einer bevorzugten Ausführungsform erfolgt die topische Anwendung in Form einer Packung, wobei die im Bereich von Heilpackungen üblichen Maßnahmen ergriffen werden können.

Die erfindungsgemäße Verwendung in Kombination mit weiteren angezeigten Maßnahmen erfolgen, beispielsweise in Kombination mit physikalischen Maßnahmen wie Bäder, Massagen oder Wärme- oder Kältebehandlungen. Solche Maßnahmen sind dem Fachmann geläufig.

In einer besonders bevorzugten Ausführungsform beinhaltet die erfindungsgemäße Verwendung, ein mit der vergorenen Enzianwurzel-Maische getränktes Vlies bei einer Temperatur von etwa 37°C bis etwa 40°C auf den Körper der Person einwirken zu lassen. Dazu wird der Körper der zu behandelnden Person mit einem Vlies umwickelt, das zuvor mit der Enzianwurzel-Maische getränkt worden ist. Zur leichteren und saubereren Handhabung kann darüber eine Schutzfolie ausgebreitet werden. Schließlich wird Wärme zugeführt, beispielsweise mit Hilfe eines oder mehrerer mit warmen Wasser befüllter Beutel oder durch Umhüllen mit einer Wärmematte. Wasserbeutel oder Wärmematte haben den zusätzlichen Vorteil, dass ein leichter Druck auf das Vlies ausgeübt wird, um auf diese Weise den Kontakt der Maische mit dem Körper der zu behandelnden Person zu verbessern. Bevorzugt ist es hierbei, wenn das Wasser der Beutel zur Steigerung des angenehmen Gefühls ungefähr Körpertemperatur hat, wobei "ungefähr Körpertemperatur" in einem Bereich von etwa 35°C bis etwa 40°C liegt, und besonders bevorzugt, wenn die Temperatur dabei bei oder geringfügig über der Körpertemperatur, also von etwa 37°C bis etwa 40°C, liegt.

Alternativ kann es auch angezeigt sein, dass die erfindungsgemäße Behandlung ohne externe Zufuhr von Wärme erfolgt, beispielsweise bei Personen, die an akuten Entzündungen leiden. Hierbei dient das bloße Umhüllen der Person, die vom Körper abgegebene Wärme zu halten.

In einer bevorzugten Ausführungsform erfolgt die Anwendung auf einem speziell für Heilpackungen entwickelten und unter dem Handelsnamen Soft Pack System® (erhältlich von der Firma Haslauer GmbH, Mitterfelden) vertriebenen System, einer Art Wasserbett. Ein solches System ist beispielsweise in der DE 103 06 280 A1 beschrieben. Auf die darin beschriebene Wanne wird in vollem Umfang Bezug genommen, womit besagte Wanne und deren Ausgestaltungen wie in der DE 103 06 280 A1 beschrieben Teil der vorliegenden Offenbarung sind. Bei dieser Art von Anwendung liegt die zu behandelnde und bereits in das Vlies eingewickelte, gegebenenfalls mit der Schutzfolie abgedeckte Person auf dem Bett, das von einer tragenden Folie überzogen ist; sobald die Hydraulik betätigt wird, steigt Wasser nach oben, und das tragende Brett sinkt nach unten. Die Person liegt nun freischwebend auf dem Wasser und wird von der Folie, die sie umhüllt, getragen.

Diese Folie, von Luft gefüllt, erwärmt sich ebenfalls, so dass die Person nicht nur von unten das erwärmte Wasser spürt, sondern gleichzeitig auch die Folie, die einen wärmenden Schutz bildet.

In einer bevorzugten Ausführungsform dauert die erfindungsgemäße Behandlung etwa 10 Minuten bis etwa 2 Stunden, bevorzugt etwa 20 Minuten bis etwa 1 Stunde, nochmals besonders bevorzugt etwa 35 Minuten bis etwa 45 Minuten. Nach jeder Behandlung wird die Person von der Maische befreit, abgetrocknet und anschließend ohne zwischenzeitliches Abduschen zur Nachtruhe gebettet.

Die Wirkung der erfindungsgemäßen Behandlung wird insgesamt als angenehm und das Wohlbefinden über die unmittelbare Behandlungsdauer hinaus verbessernd empfunden, speziell als entspannend, entstauend und die Haut glättend.

Die Erfindung wird nun anhand der folgenden Beispiele näher erläutert.

### Referenzbeispiel 1: Herstellung vergorener Enzianwurzel-Maische

4000 kg frisch geerntete Wurzeln von Gentiana lutea, gärtnerisch angebaut in O-berbayern, wurden von Hand geerntet, mit Wasser von anhaftenden Erd- und Gesteinspartikeln befreit und mit einem Häcksler mechanisch zerkleinert. Nach der Zerkleinerung wurden sie in einem Stahl-Gärbottich mit 8000 l Wasser und 75 kg Bäckerhefe versetzt und etwa 6 Wochen bei einer Temperatur, die konstant bei 30°C gehalten wurde, zur Vergärung inkubiert. Der Abschluss der Gärung war daran zu erkennen, dass keine CO₂-Entwicklung mehr stattfand. Man verwendete entweder die so erhaltene vergorene Enzianwurzel-Maische (ungebrannte vergorene Enzianwurzel-Maische), oder enzog ihr zunächst durch Destillation den Großteil ihres Alkoholgehaltes unter Produktion von 500 l Schnaps und verwendete die nach diesem Abbrennen übriggebliebene Schlempe (gebrannte vergorene Enzianwurzel-Maische) .

### Beispiel 1: Anwendung bei verschleißbedingten Schmerzen

Es kam die in Referenzbeispiel 1 beschriebene gebrannte Enzianwurzel-Maische zur Anwendung.

Die Testperson (männlich) litt vor der Behandlung seit etwa sechs Monaten unter lokalen Schmerzen in Wirbelsäule, Knie und Schultern.

Die Testperson wurde in ein Vlies von 200 cm x 160 cm gehüllt, das in ca. 1 l der Enzianwurzel-Maische getränkt war. Über dieses Vlies wurde eine Schutzfolie (170 x 200 cm) gebreitet. Dabei lag die Testperson auf einem speziell für Heilpackungen entwickelten Soft-Pack-System® (erhältlich von der Firma Haslauer GmbH, Mitterfelden), mit Hilfe dessen der Körper der Person temperiert von allen Seiten umhüllt wurde, damit sich die Wärme gleichmäßig ausbreiten konnten. Die Temperatur wurde für die Dauer der Behandlung aufrechterhalten. Alternativ erfolgte die Behandlung mittels einer Wärmematte auf einer kosmetischen Liege. Insgesamt wurden sechs Behandlungen an aufeinanderfolgenden Tagen durchgeführt, wobei jede Behandlung ca. 35 - 45 Minuten dauerte. Nach jeder Behandlung wurde die Person von der Maische befreit, abgetrocknet und anschließend zur Nachtruhe auf eine warme Gesundheitsliege gebettet. Wichtig war hierbei, vor der Nachtruhe nicht mehr zu duschen, um den Wirkstoffen die Möglichkeit zur längeren Einwirkung zu geben.

Die subjektive Wirkung der einzelnen Behandlungen war wie folgt:
1. Tag: Guter Schlaf, Gefühl von "Leichtigkeit"
2. Tag: Nachlassen der Schmerzen
3. Tag: Starke Müdigkeit
4. Tag: Müdigkeit
5. Tag: Müdigkeit, Nachlassen der Schmerzen
Nach der Behandlung beschrieb die Testperson ein leichteres Körpergefühl und Nachlassen der Schmerzen.

Die laborärztliche Untersuchung zeigte eine Senkung des Harnsäurespiegels von 8,7 mg/dl auf 7,5 mg/dl (Normbereich: bis 7,0 mg/dl).

### Beispiel 2: Anwendung bei Gicht

Die Testperson (männlich) litt seit etwa 20 Jahren unter gichtbedingten Zehenschmerzen, die in starken Phasen mit Säureantiphlogistika behandelt wurden.

Die Anwendung erfolgte wie in Beispiel 1 beschrieben. Die subjektive Wirkung der einzelnen Behandlungen war wie folgt:
1. Tag: Noch nicht schmerzfrei
2. - 6. Tag: Schmerzfrei
Nach der Behandlung beschrieb die Testperson ein gutes Gefühl und Nachlassen der Schmerzen.

### Beispiel 3: Anwendung bei Arthrose

Die Testperson (weiblich) litt seit etwa 6 Jahren unter Arthroseschmerzen in den Knien, die mit Säureantiphlogistika behandelt wurden, sowie Schmerzen im Zwölffingerdarm.

Die Anwendung erfolgte wie in Beispiel 1 beschrieben. Die subjektive Wirkung der einzelnen Behandlungen war wie folgt:
1. Tag: allgemeines Wohlgefühl
2. Tag: allgemeines Wohlgefühl
3. Tag: Nachlassen der Schmerzen, Regulierung der Verdauung
4. Tag: weiteres Nachlassen der Schmerzen, Schmerzmittel abgesetzt
5. Tag: leichteres Gefühl in den Füßen, beim Gehen Knieschmerzen
6. Tag: gute Besserung an Knien und Füßen
Nach der Behandlung beschrieb die Testperson Linderung der Schmerzen an Knien und Füßen und Verschwinden der Schmerzen im Darm.

Die laborärztliche Untersuchung zeigte Senkung der Harnsäure- und Triglyzerid-Blutspiegel sowie unkritische Veränderungen in Zellzahlen und Cholesterinspiegel.

### Beispiel 4: Anwendung bei undifferenzierten Schmerzen

Die Testperson (weiblich) litt seit etwa 25 Jahren unter nicht diagnostizierbaren Schmerzen in den Knien und der Hals- und Brustwirbelsäule, die mit Calcium und Vitamin D behandelt wurden. Die Anwendung erfolgte wie in Beispiel 1 beschrieben.

Die subjektive Wirkung der einzelnen Behandlungen war wie folgt:
1. Tag: starke Erschöpfung
2. Tag: allgemeines Wohlgefühl
3. Tag: starke Schmerzen
4. Tag: Nachlassen der Schmerzen, gute Nachtruhe
5. Tag: Weiteres Nachlassen der Schmerzen, gute Nachtruhe

Nach der Behandlung beschrieb die Testperson ihren Gesamtzustand als schmerzfrei.

Die laborärztliche Untersuchung zeigte Senkung des Cholesterinspiegels sowie unkritische Veränderungen in Zucker- und Elektrolytspiegeln.

### Beispiel 5: Anwendung bei Gelenkrheumatismus

Die Testperson (weiblich) litt unter Gelenkrheumatismus. Die Anwendung erfolgte wie in Beispiel 1 beschrieben.

Die subjektive Wirkung der einzelnen Behandlungen umfasste Verschwinden der Schmerzen innerhalb von 4 Tagen. Die laborärztliche Untersuchung zeigte nur marginale Veränderungen.

### Beispiel 6: Anwendung bei Verschleißschmerzen (II)

Die Testperson (männlich) litt unter verschleißbedingten Schmerzen in Rücken und Schultern. Die Anwendung erfolgte wie in Beispiel 1 beschrieben.

Die subjektive Wirkung der einzelnen Behandlungen war wie folgt:
2. Tag: Nachlassen der Schmerzen
3. Tag: Fast schmerzfrei
Nach der Behandlung beschrieb die Testperson ihren Gesamtzustand als angenehm und leicht.

### Beispiel 7: Anwendung bei Gicht (II)

Die Testperson (weiblich) litt unter anfallsweisen gichtbedingten Schmerzen im gesamten Körper. Die Anwendung erfolgte wie in Beispiel 1 beschrieben.

Die subjektive Wirkung der einzelnen Behandlungen war wie folgt:
1. Tag: starke Erschöpfung, gute Nachtruhe
4. Tag: Fast schmerzfrei, gute Erholung
Nach der Behandlung beschrieb die Testperson ihren Gesamtzustand als sehr gut.

### Beispiel 8: Anwendung bei entzündlichen Schmerzen

Die Testperson (weiblich) litt im Zusammenhang mit einer Hausstaub- und Eiweißallergie unter wechselnden Schmerzen in Hand- und Fußgelenken, sowie permanenten Kopfschmerzen und Sodbrennen infolge von Übersäuerung. Die Anwendung erfolgte wie in Beispiel 1 beschrieben, abgesehen davon, dass anstelle der gebrannten Enzianwurzel-Maische (Schlempe) die ungebrannte Maische aus Referenzbeispiel 1 verwendet wurde.

Die subjektive Wirkung der einzelnen Behandlungen war wie folgt:
1. Tag: Verstärkung der Kopfschmerzen und des Sodbrennens
2. Tag: Gute Nachtruhe
3. Tag: Sehr gute Nachtruhe
4. Tag: Juckreiz, kein Sodbrennen mehr

Nach der Behandlung beschrieb die Testperson Verschwinden des Sodbrennens, bessere Verdauung und Verbesserung der Nachtruhe. Weiterhin beobachtete sie eine positive Wirkung auf ihre Haut (weicher, glatter).

### Beispiel 9: Anwendung bei Arthrose (II)

Die Testperson (männlich) litt unter Arthroseschmerzen in den Fingergelenken, mit Steifheit und Verkrampfung begleitet. Behandlung geschah durch Softlaser und Vitamin E.

Die Anwendung erfolgte wie in Beispiel 8 beschrieben. Die subjektive Wirkung der einzelnen Behandlungen war wie folgt:
1. Tag: Nachlassen der Gelenksteifigkeit, angenehmes Gefühl, Entspannung
2. Tag: Wohlgefühl, Entspannung, Nachlassen der Fingerschwellungen.
Nach der Behandlung beschrieb die Testperson ihren Gesamtzustand als entspannt und erholt und die Behandlung insgesamt als schmerzlindern und abschwellend.

Die laborärztliche Untersuchung zeigte Anstieg des Triglyceridspiegels und diverse unkritische Veränderungen.

### Beispiel 10: Anwendung bei Gelenkrheumatismus (II)

Die Testperson (weiblich) litt seit 13 Jahren unter starken, zum Teil mit erheblichen Schwellungen verbundenen Schmerzen in allen größeren Gelenken, die bis zur Unbeweglichkeit führten. Behandlung geschah längere Zeit durch Cortison, vor über einem Jahr abgesetzt.

Die Anwendung erfolgte wie in Beispiel 1 beschrieben. Die subjektive Wirkung der einzelnen Behandlungen war wie folgt:
1. Tag: Schmerzfrei, ausgeprägtes Wohlbefinden, imstande zu anstrengenden manuellen Tätigkeiten
2. Tag: Abschwellung, Schmerzfreiheit, bessere Beweglichkeit
3. Tag: Zunahme der Beweglichkeit, weiterer Rückgang der Schwellungen
4. Tag: Zunahme der Beweglichkeit, weiterer Rückgang der Schwellungen
5. Tag: Weiterer Rückgang der Schwellungen
6. Tag: Leichteres Gehen, weiterer Rückgang der Schwellungen, Schmerzlinderung

Die Anwendung wurde insgesamt 3 weitere Wochen fortgesetzt, allerdings in Anlehnung an Beispiel 8. Im Laufe dieser Zeit beschrieb die Testperson ihren physischen und psychischen Gesamtzustand als dauerhaft verbessert. Insbesondere litt sie nicht mehr unter nächtlichen Krämpfen und stabilisierte sich emotional.

Die laborärztliche Untersuchung zeigte Anstieg des leichten Anstieg des Cholesterinspiegels, der Thrombozytenzahl und diverse unkritische Veränderungen.

### Beispiel 11: Anwendung bei Arthrose (III)

Die Testperson (weiblich) litt seit etwa 6 Jahren unter Arthroseschmerzen in Knien und Füßen, die bei Belastung mit Voltaren behandelt wurden. Die Anwendung erfolgte wie in Beispiel 8 beschrieben.

Die subjektive Wirkung der einzelnen Behandlungen war wie folgt:
1. Tag: allgemein angenehmes Gefühl
2. Tag: nächtliche Hitze
3. Tag: Nachlassen der Schmerzen
Nach der Behandlung beschrieb die Testperson Verschwinden der Schmerzen.

Die laborärztliche Untersuchung zeigte eine Senkung des Harnsäurespiegels von 5,2 mg/dl auf 4,7 mg/dl (Normbereich: bis 7,0 mg/dl).

### Beispiel 12: Anwendung bei undifferenzierten Schmerzen (II)

Die Testperson (weiblich) litt seit etwa 1 Jahr unter wechselnden undiagnostizierbaren Schmerzen in den großen Gelenken, die nicht medikamentös behandelt wurden. Die Anwendung erfolgte wie in Beispiel 8 beschrieben.

Die subjektive Wirkung der einzelnen Behandlungen war wie folgt:
1. Tag: allgemein angenehmes Gefühl
2. Tag: Nachlassen der Schmerzen
3. Tag: Schmerzfrei
4. Tag: Schmerzfrei
5. Tag: Schmerzfrei
6. Tag: Schmerzfrei
Nach der Behandlung beschrieb die Testperson Verschwinden der Schmerzen.

Die laborärztliche Untersuchung zeigte eine starke Sekund des C-reaktiven Proteins (CRP) von 11,7 auf 4,7 mg/l (Normbereich: bis 5,0 mg/l).

### Beispiel 13: Anwendung bei unfallbedingten Schmerzen

Die Testperson (weiblich) litt seit etwa 13 Jahren infolge eines Unfalls unter Schmerzen in Füßen, Knien und Schulter, die nicht medikamentös behandelt wurden. Die Anwendung erfolgte wie in Beispiel 8 beschrieben.

Die subjektive Wirkung der einzelnen Behandlungen war wie folgt:
1. Tag: allgemein angenehmes Gefühl, Müdigkeit
2. Tag: Gute Nachtruhe, starker Harndrang
3. Tag: Schmerzfrei
4. Tag: Schmerzfrei
5. Tag: Schmerzfrei
6. Tag: Schmerzfrei
Nach der Behandlung beschrieb die Testperson Verschwinden der Schmerzen.

### Beispiel 14: Anwendung auf dem Wasserbett bei Verschleißschmerzen (III)

Die Testperson (weiblich) litt seit mehreren Wochen unter Schmerzen in Händen und Schultern, die mit Säureantiphlogistika behandelt wurden, sowie nächtlichen Krämpfen in den Füßen. Die Anwendung erfolgte wie in Beispiel 8 beschrieben.

Die subjektive Wirkung der einzelnen Behandlungen umfasste Nachlassen der Schmerzen und Verbesserung der Nachtruhe ab dem dritten Behandlungstag. Nach der Behandlung beschrieb die Testperson Verschwinden der Krämpfe und Linderung der Schmerzen.

### Beispiel 15: Anwendung bei undifferenzierten Schmerzen (III)

Die Testperson (weiblich) litt seit etwa 3 Jahren unter wechselnden undiagnostizierbaren Schmerzen in Händen und Füßen, die nicht medikamentös behandelt wurden. Die Anwendung erfolgte wie in Beispiel 8 beschrieben.

Die subjektive Wirkung der einzelnen Behandlungen umfasste Nachlassen der Schmerzen ab dem dritten und Schmerzfreiheit ab dem vierten Behandlungstag. Nach der Behandlung beschrieb die Testperson Schmerzfreiheit und Rückbildung ihrer Krampfadern, außerdem eine positive Wirkung auf ihre Haut (weicher, glatter).

## Patentansprüche

1. Pharmazeutisches oder kosmetisches topisches Mittel, **gekennzeichnet durch** einen Gehalt an vergorener Enzianwurzel-Maische.

2. Mittel gemäß Anspruch 1, **dadurch gekennzeichnet, dass** die vergorene Enzianwurzel-Maische erhältlich ist, indem man Enzianwurzeln mechanisch aufschließt, mit Wasser versetzt, vergärt und gegebenenfalls wenigstens einen Teil des gebildeten Alkohols abtrennt.

3. Mittel gemäß Anspruch 2, **dadurch gekennzeichnet, dass** die vergorene Enzianwurzel-Maische die aus dem Gärungsprozess vor dem Abtrennen des gebildeten Alkohols erhältliche Maische ist.

4. Mittel gemäß Anspruch 2, **dadurch gekennzeichnet, dass** die vergorene Enzianwurzel-Maische die nach Abbrennen wenigstens eines Teils des gebildeten Alkohols erhältliche Maische ist.

5. Verwendung einer vergorenen Enzianwurzel-Maische zur Erhöhung des Wohlbefindens einer Person.

6. Verwendung nach Anspruch 5, wobei die Erhöhung des Wohlbefindens eine Milderung eines unspezifischen Erschöpfungs- und/oder Unwohlseinszustandes ist.

7. Verwendung nach Anspruch 5, wobei die Erhöhung des Wohlbefindens eine als entspannend, entstauend und/oder die Haut glättend empfundene Wirkung ist.

8. Verwendung einer vergorenen Enzianwurzel-Maische zur Linderung von Beschwerden einer Person.

9. Verwendung nach Anspruch 8, wobei die Beschwerden ausgewählt sind unter Stoffwechselstörungen, bevorzugt Gicht; Beschwerden im Nieren- und Harnwegssystem; Verletzungen, bevorzugt Prellungen, Verstauchungen, Verbrennungen und Insektenstichen; Befindlichkeitsstörungen, bevorzugt Schlafstörungen, Verspannungen, Nervosität und chronischen und/oder undefinierten Schmerzen; entzündlichen Vorgängen, bevorzugt Rheuma, Arthrose, Arthritiden, Phlebitiden, chronischen Darmentzündungen und Multipler Sklerose; Hauterkrankungen, bevorzugt Neurodermitis; und Lymphstauungen.

10. Verwendung nach einem der Ansprüche 5 bis 9 in Form einer topischen Anwendung.

11. Verwendung nach Anspruch 10, wobei die topische Anwendung eine Packung ist.

12. Verwendung nach Anspruch 10, wobei die topische Anwendung umfasst, ein mit der vergorenen Enzianwurzel-Maische getränktes Vlies bei einer Temperatur von etwa 37°C bis etwa 40°C etwa 10 Minuten bis etwa 2 Stunden auf den Körper der Person einwirken zu lassen.
